# EUROPEAN PATENT APPLICATION

(11) **EP 4 389 732 A1**
(43) Date of publication of application: **26.06.2024**
(21) Application number: 22857842.3
(22) Date of filing: 17.08.2022
(51) Int. Cl.: C07C 49/613, C07C 13/28, C07C 233/15, C07D 491/22

(54) **METHOD FOR SYNTHESIZING 5,8-DIAMINO-3,4-DIHYDRO-2H-1-NAPHTHALENONE AND INTERMEDIATE COMPOUND USED THEREIN**

(30) Priority: 17.08.2021 CN 202110943248
(71) Applicant: Jiangsu Mabwell Health Pharmaceutical R&D Co., Ltd., China Medical City Taizhou, Jiangsu 225300 (CN); Mabwell (Shanghai) Bioscience Co., Ltd., Shanghai 201210 (CN)
(72) Inventor: XU, Hui, Taizhou City, Jiangsu 225300 (CN); ZHOU, Wei, Taizhou City, Jiangsu 225300 (CN); WANG, Zhenzhen, Taizhou City, Jiangsu 225300 (CN); TAN, Xiaoding, Taizhou City, Jiangsu 225300 (CN)
(74) Representative: Schiweck Weinzierl Koch Patentanwälte Partnerschaft mbB
(86) International application number: PCT/CN2022/112985
(87) International publication number: WO 2023/020531

(57) **Abstract**

A method for synthesizing 5,8-diamino-3,4-dihydro-2H-1-naphthalenone (Compound I), which method comprises: subjecting 2,5-diprotected aminophenylbutyric acid to the Friedel-Crafts reaction for ring closure, and then removing a protecting group on the amino group to obtain Compound I.

## Description

### CROSS-REFERENCE TO RELATED APPLICATION(S)

The present patent application claims priority to and the benefit of Chinese patent application No. 202110943248.1 filed on August 17, 2021, which is incorporated herein by reference in its entirety.

### TECHNICAL FIELD

The present invention belongs to the field of preparation of pharmaceutical intermediates, and particularly relates to a synthetic method of 5,8-diamino-3,4-dihydro-2H-1 -naphthalenone which is a starting material for the preparation of camptothecins, as well as intermediate compounds used in the method.

### BACKGROUND OF THE INVENTION

As topoisomerase I inhibitors, camptothecins (CPTs) can specifically inhibit DNA topoisomerase I and hinder the closing of DNA chains, leading to broken DNA single-strands which cannot be reconnected and finally leading to cell death, and are one class of important anti-tumor drugs.

Meanwhile, the compounds are also widely used in preparing antibody-conjugated drugs (ADCs). In ADCs, camptothecins are used as the small molecule drugs exerting antitumor activity and are coupled with antibodies or antibody-like ligands to form targeted therapeutic drugs. For example, Sacituzumab govitecan (IMMU-132) is formed from a camptothecin compound SN38 coupling with an anti-Trop-2 antibody, and Enhertu (DS-0821) is formed from Exatecan coupling with Trastuzumab.

In patent publication document WO2020200880A1, an ADC of the following structure is reported: 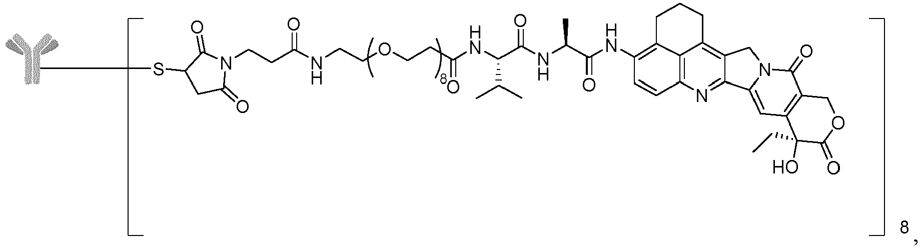 in which the camptothecin compound CPT-1 used has the following structure:

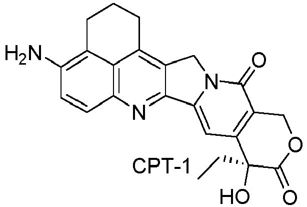

As report in WO2020200880A1 and a literature (Journal of Medicinal Chemistry, 1998, vol. 41, #13, p.2308-2318), CPT-1 can be obtained by the condensation of 5,8-diamino-3,4-dihydro-2H-1-naphthalenone (Compound I) with Compound II: 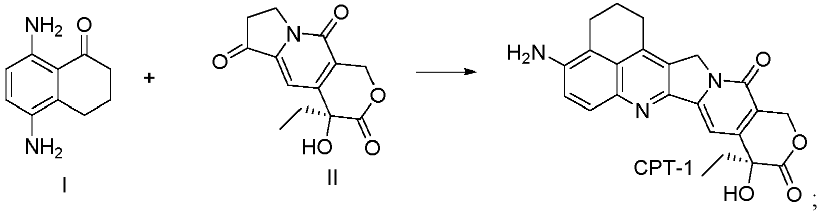 however, the starting material Compound I for synthesizing CPT-1 is required to be provided via the following routes:
synthetic route 1 (the overall reaction yield would be 24.2%): 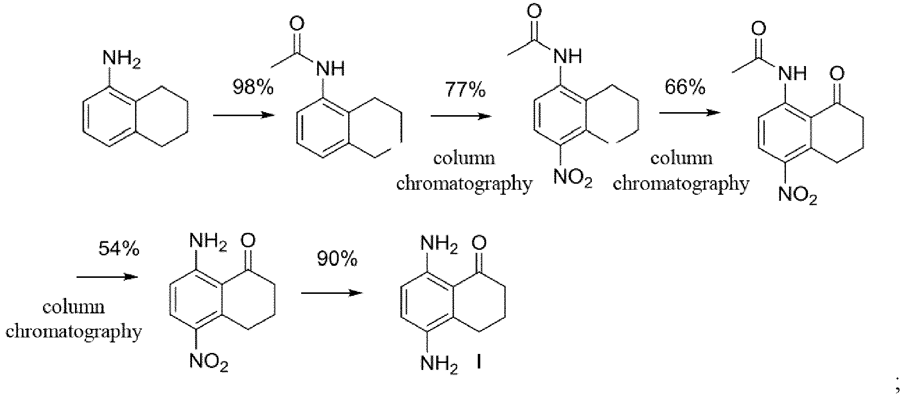
synthetic route 2 (the overall reaction yield would be 15.6%): 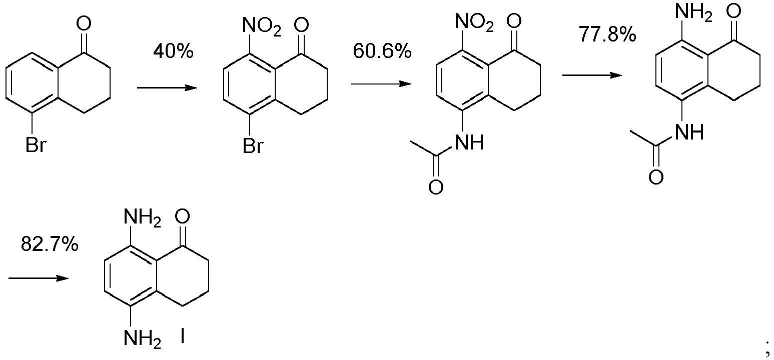
synthetic route 3 (the overall reaction yield would be 33.2%):

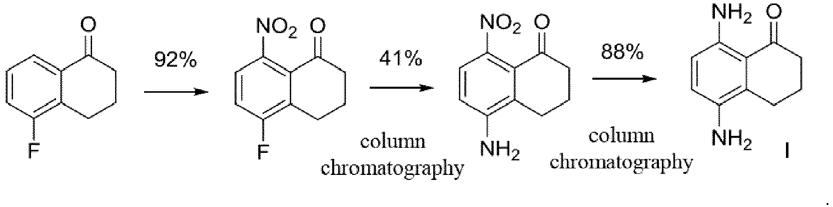

As shown above, in addition to the rather low overall reaction yield obtain by any one of the above synthetic routes, all the routes comprise a nitration process, which may cause isomers to appear, and employ multiple column chromatography steps for purification, which make the operation of the whole process complicated and may have some risks when the production scale is enlarged; meanwhile, all the synthetic routes require post-treatment operations or have high requirements on equipment, and accordingly are not suitable for industrial production.

### SUMMARY OF THE INVENTION

The technical problem to be solved by the present invention is to provide a new synthetic method of 5,8-diamino-3,4-dihydro-2H-1-naphthalenone (Compound I), to circumvent the reactions and operations in the existing synthetic routes, such as nitration, oxidation and the like, which are not suitable for scale-up production of Compound I. The method shall realize industrial production of Compound I, so that starting material compounds for synthesizing the camptothecin compound CPT-1 and even ADCs can be better provided.

In order to solve the above technical problem, one object of the present disclosure is to provide an intermediate compound having a novel chemical structure for synthesizing Compound I, and in turn synthesizing the camptothecin compound CPT-1; in addition, is to provide a new synthetic method of Compound I using the intermediate compound as a starting material.

The present disclosure provides the following technical solutions.

In one aspect, the present disclosure provides a synthetic method of 5,8-diamino-3,4-dihydro-2H-1-naphthalenone i.e. Compound I, the synthetic method comprising: subjecting 2,5-di-protected aminophenylbutyric acid of formula III to a Friedel-crafts reaction to achieve ring closing, and removing the protecting groups on the amino groups to obtain Compound I, 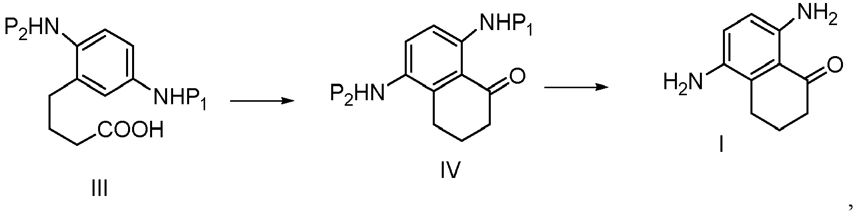 wherein P₁ and P₂ are amino protecting groups.

Preferably, P₁ and P₂ are independently selected from the group consisting of acetyl, benzoyl, benzyloxycarbonyl (Cbz) and fluorenylmethyloxycarbonyl (Fmoc); more preferably, P₁ and P₂ are the same; further preferably, both P₁ and P₂ are acetyl.

In the synthetic method, the Friedel-crafts reaction is carried out under the catalysis by polyphosphoric acid; or the Friedel-crafts reaction is carried out by reacting 2,5-di-protected aminophenylbutyric acid of formula III with oxalyl chloride or sulfoxide chloride to prepare an acyl chloride intermediate and then performing catalysis by aluminum trichloride.

In the synthetic method provided by the present disclosure, 2,5-di-protected aminophenylbutyric acid of formula III is provided via the following route 1 (palladium-catalyzed coupling with zinc reagent):
synthetic route 1: 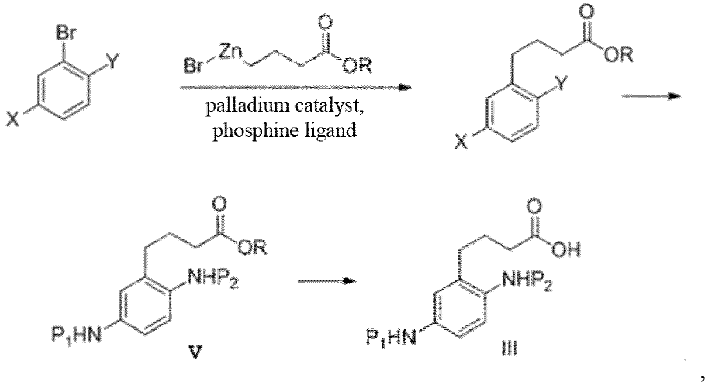 wherein X and Y are independently selected from the group consisting of nitro, amino, or P₁ or P₂ protected amino; R is a C₁-C₆ alkyl or benzyl, preferably selected from the group consisting of methyl, ethyl, isopropyl, tert-butyl and benzyl, more preferably methyl or ethyl.

Preferably, the palladium catalyst is selected from the group consisting of palladium acetate, palladium chloride, dppf palladium dichloride, Tetrakis(triphenylphosphine)palladium, and the like, more preferably palladium acetate. Preferably, the phosphine ligand is selected from the group consisting of BINAP, S-PHOS and X-PHOS, more preferably S-PHOS.

According to one particular embodiment of the present disclosure, synthetic route 1 comprises:
1) when X is amino and Y is nitro: 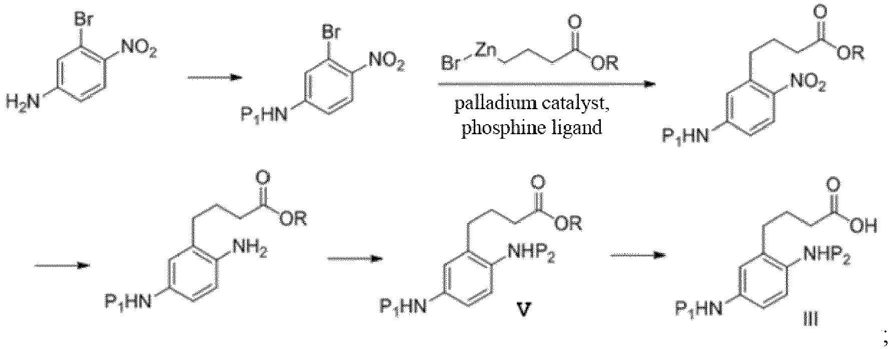
2) when both X and Y are amino: 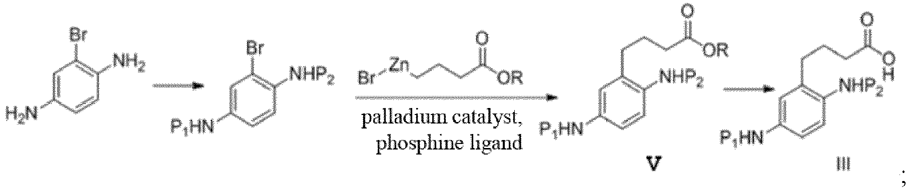 or
3) when X is nitro and Y is amino:

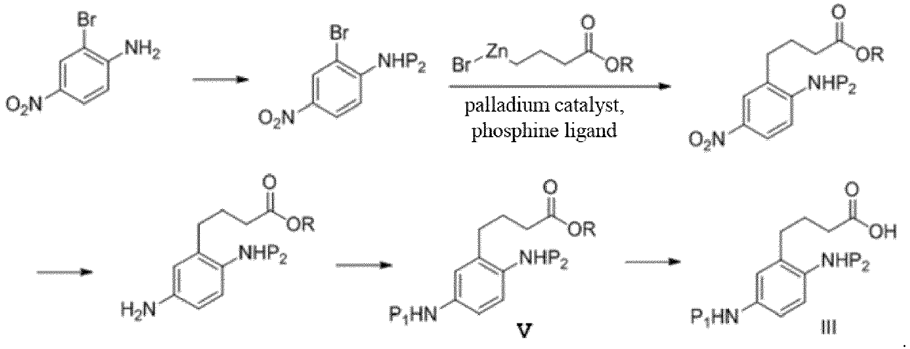

Alternatively, 2,5-di-protected aminophenylbutyric acid of formula III is provided via the following route 2 (amide hydrolysis):
synthetic route 2: 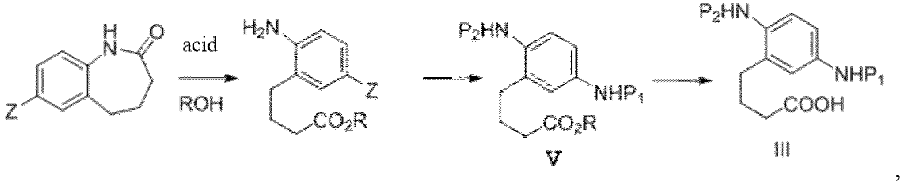
in above synthetic route 2, Z is nitro, amino, P₁ or P₂ protected amino; R is a C₁-C₆ alkyl or benzyl, preferably selected from the group consisting of methyl, ethyl, isopropyl, tert-butyl and benzyl, more preferably methyl or ethyl.

Preferably, the acid is selected from the group consisting of sulfuric acid, hydrogen chloride, sulfoxide chloride and p-toluenesulfonic acid, more preferably sulfuric acid.

According to one particular embodiment of the present invention, synthetic route 2 comprises:
1) when Z is amino: 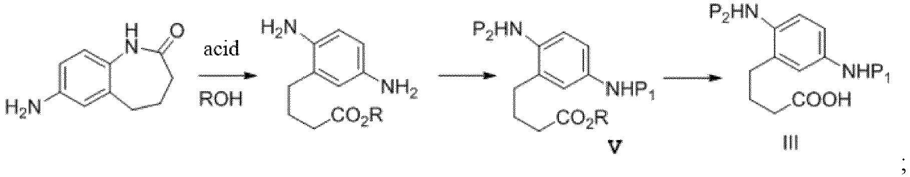
2) when Z is nitro: 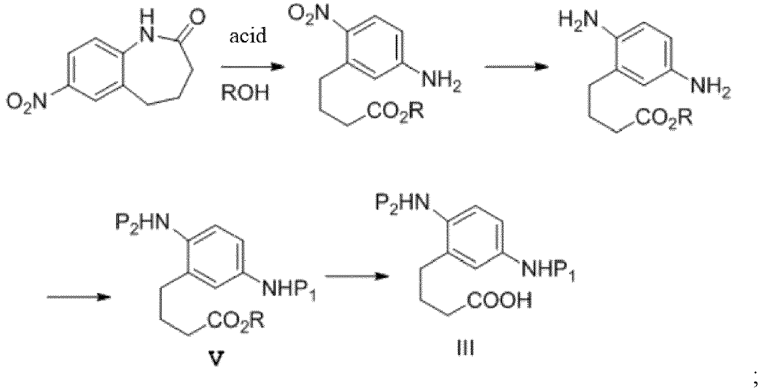 or

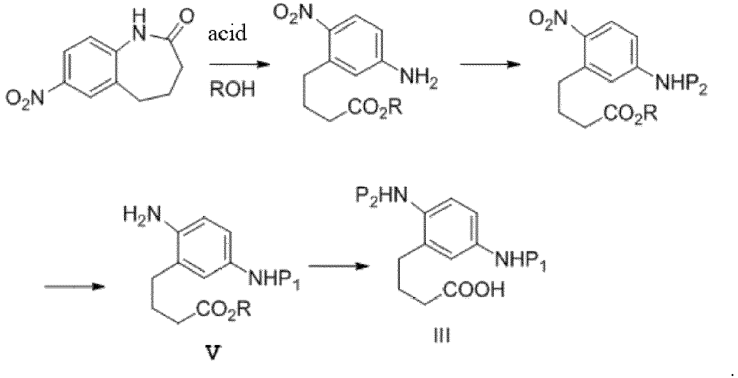

In another aspect, the present disclosure provides an intermediate compound used in the synthetic method of 5,8-diamino-3,4-dihydro-2H-1-naphthalenone i.e. Compound I, including as follows.

The present disclosure provides a compound of formula V: 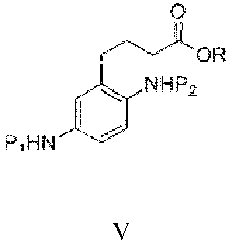 wherein P₁ and P₂ are amino protecting groups; and R is a C₁-C₆ alkyl or benzyl.

Preferably, P₁ and P₂ are independently selected from the group consisting of acetyl, benzoyl, benzyloxycarbonyl (Cbz) and fluorenylmethyloxycarbonyl (Fmoc); more preferably, P₁ and P₂ are the same; further preferably, both P₁ and P₂ are acetyl.

Preferably, R is selected from the group consisting of methyl, ethyl, isopropyl, tert-butyl and benzyl, more preferably methyl or ethyl.

According to one particular embodiment of the present disclosure, the compound of formula V
is compound D:

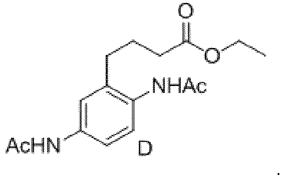

The present disclosure provides a compound of formula III: 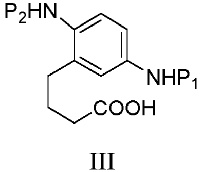 wherein, P₁ and P₂ are amino protecting groups.

Preferably, P₁ and P₂ are independently selected from the group consisting of acetyl, benzoyl, benzyloxycarbonyl (Cbz) and fluorenylmethyloxycarbonyl (Fmoc); more preferably, P₁ and P₂ are the same; further preferably, both P₁ and P₂ are acetyl.

According to one particular embodiment of the present disclosure, the compound of formula III 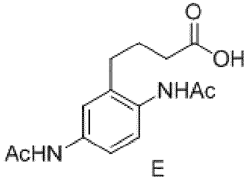 is compound E:
In a further aspect, the present disclosure provides use of the compound of formula V and/or the compound of formula III for the preparation of Compound I, camptothecins and/or an antibody-drug conjugate.

Compared with the prior arts, the present disclosure provides a new synthetic method of Compound I. The synthetic method uses the compound of formula V or the compound of formula III as an intermediate compound, and via a new synthetic route, achieves the synthesis of Compound I with a high yield. Further, the synthetic method circumvents the reactions and operations in the existing synthetic routes for Compound I, such as nitration, oxidation and the like, which are not suitable for scale-up production, so is more suitable for the industrial production of Compound I, thereby better providing starting material compounds for synthesizing the camptothecin compound CPT-1. In addition, the present disclosure provides intermediate compounds used in the synthetic method and accordingly, provides more alternative starting materials for synthesizing or preparing Compound I, camptothecins, and even antibody-drug conjugates.

### BRIEF DESCRIPTION OF THE DRAWINGS

Embodiments of the present invention are described in detail below with reference to the attached figures, in which:
Figure 1: ¹H NMR (D₆-DMSO) of Compound D.
Figure 2: ¹H NMR (D₆-DMSO) of Compound E.
Figure 3: ¹H NMR (D₆-DMSO) of Compound I.
Figure 4: Mass spectrum of Compound I.

### DETAILED DESCRIPTION OF PREFERRED EMBODIMENTS

The present invention is illustrated below with reference to specific examples. It will be understood by those skilled in the art that these examples are merely illustrative of the present invention and do not limit the scope of the present invention in any way.

Experimental procedures in the following examples are all conventional ones, unless otherwise specified. Raw materials and reagents used in the following examples are all commercially available products, unless otherwise specified.

### Example 1 Synthesis of the intermediate D (Synthetic route 1 - Palladium catalyzed coupling with zinc reagent)

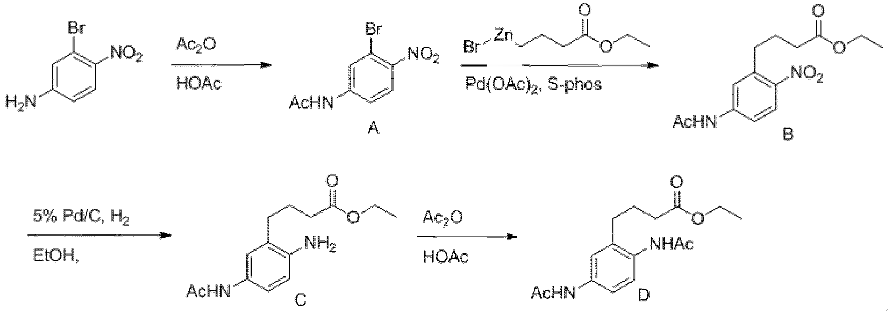

### (1) Synthesis of the intermediate A

3-bromo-4-nitroaniline (25.00 g, 0.12 mol) and 200 mL of acetic acid were added into a 500 mL flask, into which 50 mL of acetic anhydride was then slowly added dropwise. The starting material compounds reacted at room temperature for 16 hours. After the reaction completed as confirmed with TLC detection, the reaction liquid was filtered to obtain the filtrate, which was in turn concentrated under reduced pressure to remove acetic acid. The filter cakes obtained were pooled and slurried with 200 mL of MTBE, and then filtered to obtain 27.6 g of dried yellow solid (the intermediate A) with a yield of 92%. LC-MS (ESI): m/z 259 (M + H)⁺.

### (2) Preparation of 4-ethoxy-4-oxobutylzinc bromide

Activated zinc powder (19.0 g, 0.29 mol, 2.00 eq) was added into a 250 mL three-neck flask (equipped with a thermometer, reflux condenser tubes and rubber plugs). Then the air in the flask was replaced with nitrogen, anhydrous DMF (145 mL) was added, and then the air in the flask was replaced with nitrogen again. Iodine (1.86 g, 0.015mol, 0.1 eq) was added at room temperature, and the color of the solution was observed to change from colorless to brownish red, to light yellow gradually, and to colorless finally (in 2-3 min). Next, ethyl 4-bromobutyrate (28.6 g, 0.15 mol, and 1.00 eq) was added into the flask which was then heated to 80 °C (inner temperature) for reaction for 4-5 hours. When the reaction completed as confirmed with TLC detection, the reaction liquid was allowed to stand and cool to room temperature for later use. The supernatant obtained was light yellow and had a concentration of 1 mol/L.

### (3) Synthesis of the intermediate B

Anhydrous DMF (120 mL) and the intermediate A (25.0 g, 1.00 eq) were added into a 500 mL reaction flask. Then the air in the flask was replaced with nitrogen, palladium acetate (433 mg, 0.02 eq) was added, and then the air in the flask was replaced with nitrogen again. The reaction mixture in the flask was stirred at room temperature for 10 min, then S-PHOS (1.6 g, 0.04 eq) was added, and then the air in the flask was replaced with nitrogen again. Next, the reaction mixture in the flask was stirred for 20 min, 4-ethoxy-4-oxobutylzinc bromide (145 mL, 1.50 eq) prepared in the above step was added dropwise at room temperature (25-30 °C), and then the reaction was maintained at 25-30 °C for 16 hours. When the starting material compounds reacted completely as confirmed with TLC detection (DCM: EA =5: 1), the reaction liquid was cooled to room temperature, and ammonium chloride solution (15 mL) was added to the reaction liquid to quench the reaction. Then the reaction liquid was poured into 1 L of water, and 400 mL of ethyl acetate was added, to obtain separated phases. The phases were filtered through a buchner funnel, and then the aqueous phase obtained was extracted with ethyl acetate (400 mL × 2); and the organic phases obtained were washed with water (500 mL) twice and with saturated sodium chloride solution (500 mL) once, then dried over anhydrous sodium sulfate, and concentrated under reduced pressure to obtain 37.0 g of reddish brown oily liquid (the intermediate B) with a yield of 130%. The crude product was used in the next reaction without further purification. LC-MS (ESI): m/z 295 (M + H)⁺.

### (4) Synthesis of the intermediate C

The intermediate B (37.0 g, 1.0 eq) and ethanol (1.5 L) were added into a 3 L three-neck reaction flask and the starting material was completely dissolved in the ethanol. The reaction system was cooled to 5 °C, 5% Pd/C (5.7 g, 1.0 eq) was added, and the temperature was raised to 25 °C in a hydrogen atmosphere (atmospheric pressure) for reaction for 16 hours. When the reaction completed as confirmed with TLC detection (DCM: EA =5: 1), the reaction system was filtered through celite, and the organic phase obtained was concentrated to give 33.0 g of a crude product (the intermediate C) with a yield of 130%. The crude product was used in the next reaction without further purification. LC-MS (ESI): m/z 265 (M + H)⁺.

### (5) Synthesis of the intermediate D

The intermediate C (33.0 g, 1.00 eq) and 260 mL of acetic acid were added into a 1 L flask, into which 46 mL of acetic anhydride was then slowly added dropwise. The starting material compounds reacted at room temperature for 2 hours, and when reacted completely as confirmed with TLC detection (DCM: MeOH =10: 1), the reaction liquid was filtered to obtain the filtrate, which was in turn concentrated under reduced pressure to remove acetic acid. 250 mL of water was added, and then the aqueous phase obtained was extracted with ethyl acetate (400 mL × 2); and the organic phases obtained were washed with water (500 mL) twice and with saturated sodium chloride solution (500 mL) once, then dried over anhydrous sodium sulfate, and concentrated under reduced pressure to obtain 32 g of a crude product. The crude product was slurried with 400 mL of MTBE, and then filtered to obtain 27.0 g of dried light yellow solid (the intermediate D) with a yield of 91%. The total yield of the four steps was found to be 83.7%.

LC-MS (ESI): m/z 307 (M+H)⁺.

¹H NMR (400 MHz, DMSO) δ 9.87 (s, 1H), 9.19 (s, 1H), 7.41 (s, 1H), 7.37 (d, *J* = 8.7 Hz, 1H), 7.20 (d, *J* = 8.5 Hz, 1H), 4.06 (q, *J* = 7.0 Hz, 2H), 2.52 (d, *J* = 8.5 Hz, 2H), 2.29 (t, *J* = 7.3 Hz, 2H), 2.02 (s, 6H), 1.79 - 1.64 (m, 2H), 1.18 (t, *J* = 7.1 Hz, 3H).

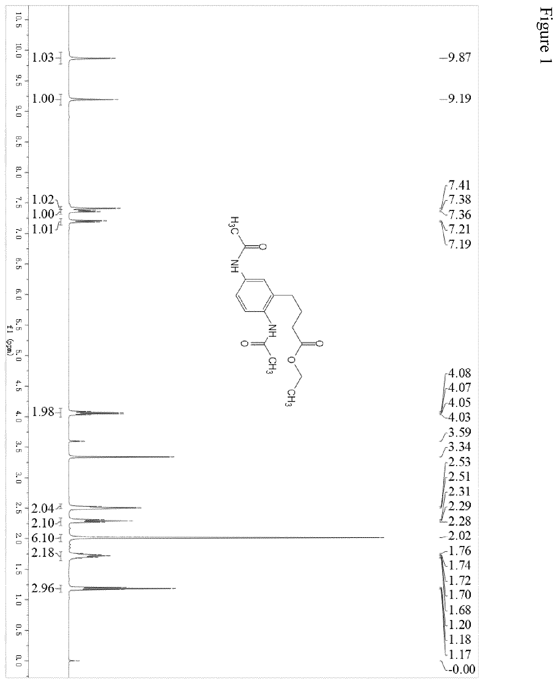

¹H-NMR spectrum is shown in Figure 1.

### Example 2 Synthesis of the intermediate D (Synthetic route 1 - Palladium catalyzed coupling with zinc reagent)

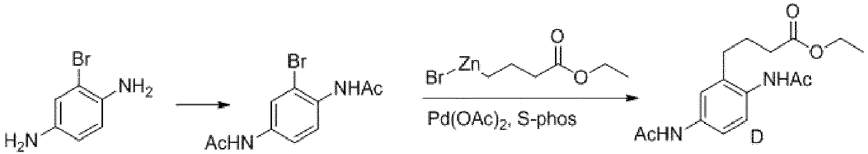

2,5-diaminobromobenzene was acetylated by a similar process to that in step (1) of Example 1 to obtain 2,5-diacetamidobromobenzene which was light yellow solid with a yield of 93%.

The zinc reagent (4-ethoxy-4-oxobutylzinc bromide) was prepared according to the process in step (2) of Example 1.

Anhydrous DMF (120 mL) and 2,5-diacetamidobromobenzene (26.1 g, 96.5mmol, 1.00 eq) were added into a 500 mL reaction flask. Then the air in the flask was replaced with nitrogen, palladium acetate (433 mg, 1.93 mmol, 0.02 eq) was added, and then the air in the flask was replaced with nitrogen again. The reaction mixture in the flask was stirred at room temperature for 10 min, then S-PHOS (1.6 g, 3.85 mmol, 0.04 eq) was added, and then the air in the flask was replaced with nitrogen again. Next, the reaction mixture in the flask was stirred for 20 min, 4-ethoxy-4-oxobutylzinc bromide (145 mL, 145 mmol, 1.50 eq) prepared in the above step was added dropwise at room temperature (25-30 °C), and then the reaction was maintained at 65-70 °C for 16 hours. When the starting material compounds reacted completely as confirmed with TLC detection, the reaction liquid was cooled to room temperature, and ammonium chloride solution (15 mL) was added to the reaction liquid to quench the reaction. Then the reaction liquid was poured into 1 L of water, and 400 mL of ethyl acetate was added, to obtain separated phases. The aqueous phase obtained was extracted with ethyl acetate (400 mL × 2); and the organic phases obtained were pooled and washed with water (500 mL) twice and with saturated sodium chloride solution (500 mL) once, then dried with anhydrous sodium sulfate, suctioned, and concentrated under reduced pressure. The crude product obtained was purified by column chromatography using an eluent (DCM: MeOH =50:1 to 30:1), and then 13.8 g of the intermediate D was obtained as light yellow solid with a yield of 46.7%. The total yield of the two steps was found to be 43.4%. LC-MS (ESI): m/z 307 (M+H)⁺.

### Example 3 Synthesis of the intermediate D (Synthetic route 1 - Palladium catalyzed coupling with zinc reagent)

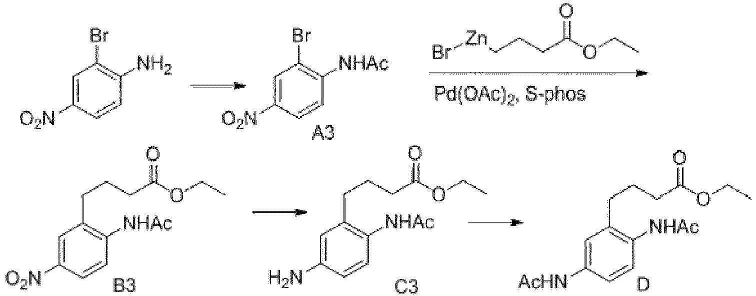

2-amino-5-nitrobromobenzene was acetylated by a similar process to that in step (1) of Example 1 to obtain 2-acetamido-5-nitrobromobenzene which was yellow solid with a yield of 90%.

The zinc reagent (4-ethoxy-4-oxobutylzinc bromide) was prepared according to the process in step (2) of Example 1.

### (1) Synthesis of the intermediate B3

Anhydrous DMF (120 mL) and 2-acetamido-5-nitrobromobenzene (25.0 g, 96.5 mmol, 0.1 mol) were added into a 500 mL reaction flask. Then the air in the flask was replaced with nitrogen, palladium acetate (433 mg, 1.93 mmol, 0.02 eq) was added, and then the air in the flask was replaced with nitrogen again. The reaction mixture in the flask was stirred at room temperature for 10 min, then S-PHOS (1.6 g, 3.85 mmol, 0.04 eq) was added, and then the air in the flask was replaced with nitrogen again. Next, the reaction mixture in the flask was stirred for 20 min, 4-ethoxy-4-oxobutylzinc bromide (145 mL, 145 mmol, 1.50 eq) prepared in the above step was added dropwise at room temperature (25-30 °C), and then the reaction was maintained at 65-70 °C for 16 hours. When the starting material compounds reacted completely as confirmed with TLC detection, the reaction liquid was cooled to room temperature, and ammonium chloride solution (15 mL) was added to the reaction liquid to quench the reaction. Then the reaction liquid was poured into 1 L of water, and 400 mL of ethyl acetate was added, to obtain separated phases. The aqueous phase obtained was extracted with ethyl acetate (400 mL × 2); and the organic phases obtained were pooled and washed with water (500 mL) twice and with saturated sodium chloride solution (500 mL) once, then dried with anhydrous sodium sulfate, suctioned, and concentrated under reduced pressure. The crude product obtained was purified by column chromatography using an eluent (DCM: MeOH =50:1 to 30:1), and then 10.3 g of the intermediate B3 was obtained as yellow solid with a yield of 36.5%. LC-MS (ESI): m/z 295 (M+H)+.

### (2) Synthesis of the intermediate C3

The intermediate B3 (10.3 g) and ethanol (250 mL) were mixed in a 500 mL reaction flask. The reaction system was cooled to 5 °C, 5% Pd/C (1.5 g, 15% w/w) was added, and the temperature was raised to 25 °C in a hydrogen atmosphere (atmospheric pressure) for reaction for 16 hours. When the reaction completed as confirmed with TLC detection (DCM: EA=5: 1), the reaction system was filtered through celite, and the organic phase obtained was concentrated to obtain 9.4 g of a crude product (the intermediate C). The crude product was used in the next reaction in the theoretical amount without further purification. LC-MS (ESI): m/z 265 (M+H)+.

### (3) Synthesis of the intermediate D

The intermediate C3 (9.4 g, 1.00 eq) and 260 mL of acetic acid were added into a 1 L flask, into which 46 mL of acetic anhydride was then slowly added dropwise. The starting material compounds reacted at room temperature for 2 hours, and when reacted completely as confirmed with TLC detection (DCM: MeOH =10: 1), the reaction liquid was filtered to obtain the filtrate, which was in turn concentrated under reduced pressure to remove acetic acid. 150 mL of water was added, and then the aqueous phase obtained was extracted with ethyl acetate (100 mL × 2); and the organic phases obtained were pooled and washed with water (100 mL) twice and with saturated sodium chloride solution (100 mL) once, then dried with anhydrous sodium sulfate, and concentrated under reduced pressure to obtain a crude product. The crude product was slurried with 200 mL of MTBE, and then filtered to obtain 9.6 g of dried light yellow solid (the intermediate D) with a yield of 89%. The total yield of the four steps was found to be 29.2%. LC-MS (ESI): m/z 307 (M+H)+.

### Example 4 Synthesis of the intermediate D (Synthetic route 2 - Amide hydrolysis)

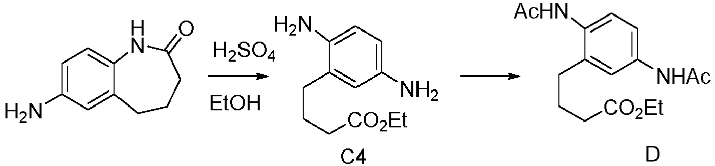

7-amino-4,5-dihydro-1H-benzo[b]azepin-2(3H)-one (17.6 g, 0.1 mmol), ethanol (250 mL), and 98% sulfuric acid (5 mL) were mixed in a 500 mL three-necked flask, which was then heated under reflux for reaction for 24 hours. The reaction liquid was sampled and detected to see whether the reaction completed. When the reaction completed, the reaction liquid was concentrated under reduced pressure to dry. Dichloromethane (200 mL) and water (100 mL) were added into the residue obtained, and the reaction mixture was placed in an ice bath, cooling to below 10 °C. 1 N sodium hydroxide aqueous solution was added to adjust the pH to 7-8, and then the mixture was stirred to obtain separated phases. The aqueous phase obtained was extracted with dichloromethane; and the organic phases were pooled and washed with saturated saline water, then dried with anhydrous sodium sulfate, suctioned, and distilled under reduced pressure to remove the organic solvent and obtain 25 g of diaminoethyl ester intermediate C4 crude product, which was directly used for the next reaction.

The intermediate C4 was dissolved in dichloromethane (200 mL), into which triethylamine (20.2 g, 0.2 mol, 2 eq) was added. The reaction mixture was placed in an ice bath, cooling to below 10 °C, and acetic anhydride (25.5 g, 0.25 mol, 2.5 eq) was added dropwise. After that, the temperature was maintained for reaction for 1 hour. The reaction liquid was sampled and detected to see whether the reaction completed. When the reaction completed, the reaction liquid was poured into 1 N ice-cold hydrochloric acid, and stirred for 15 min. Separated phases were obtained, and the aqueous phase obtained was extracted with dichloromethane (50 mL × 3); and the organic phases obtained were pooled and washed with saturated saline water, then dried with anhydrous sodium sulfate, suctioned, and distilled under reduced pressure to remove the organic solvent and obtain a crude product. The crude product was slurried with 400 mL of MTBE, and then filtered and dried to obtain 26.9 g of the intermediate D as light yellow solid. The total yield of the two steps was found to be 87.8%. LC-MS (ESI): m/z 307 (M+H)⁺.

### Example 5 Synthesis of the intermediate E

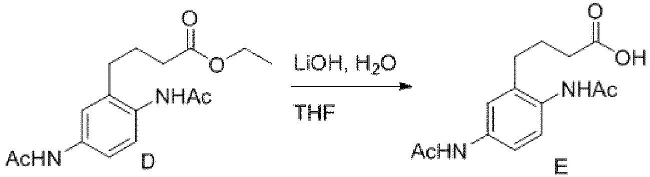

The intermediate D (27.0 g, 88 mmol, 1.0 eq), water (100 mL) and tetrahydrofuran (200 mL) were added into a 500 mL three-neck reaction flask and the starting material was completely dissolved. Lithium hydroxide monohydrate (18.5 g, 441 mmol, 5.0 eq) was added at room temperature and the reaction system was maintained at room temperature for reaction for 3 hours. When the reaction completed as confirmed with TLC detection, most of tetrahydrofuran was distilled off under reduced pressure, and 300 mL of water were added to the residue. The aqueous phase obtained was extracted with EA (2 × 100 mL) and the organic phases obtained were discarded. The aqueous phases obtained were pooled and placed in an ice bath and adjusted to pH 4 with the addition of 6 N hydrochloric acid. Solid precipitated and was filtered to obtain 19.1 g of the intermediate E as white solid with a yield of 78%.

1H NMR (400 MHz, DMSO) δ 12.09 (s, 1H), 9.86 (s, 1H), 9.18 (s, 1H), 7.38 (d, J = 15.5 Hz, 2H), 7.23 (d, J = 8.5 Hz, 1H), 2.51 (d, J = 8.1 Hz, 2H), 2.23 (t, J = 7.1 Hz, 2H), 2.02 (s, 6H), 1.76 - 1.61 (m, 2H).

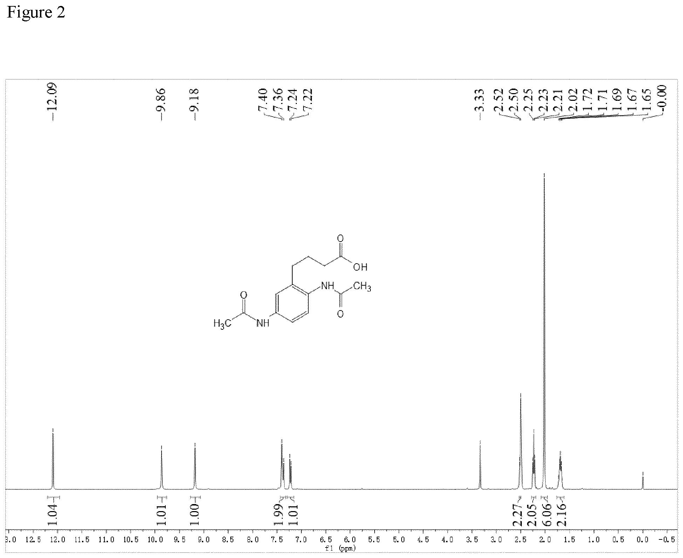

¹H-NMR spectrum is shown in Figure 2.

### Example 6 Synthesis of Compound I (Ring-closing with polyphosphoric acid)

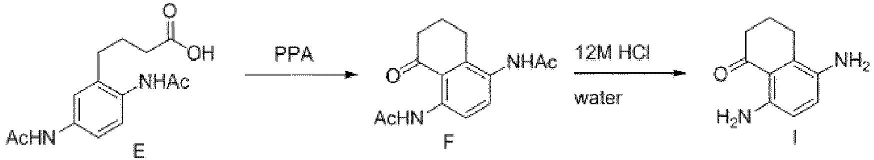

40 mL of polyphosphoric acid was added to a 250 mL reaction flask, which was then heated to 90 °C and into which the intermediate E (5.0 g, 17.97 mmol) was added in several parts. The internal temperature was maintained at 95-100 °C for reaction for 5 hours. When the reaction completed as confirmed with TLC detection, heating source was removed, and the reaction system was allowed to cool to 50-60 °C. Next, 15 mL of 4 M HCl (aq) was added dropwise into the reaction system to quench the reaction (the temperature would raise to 100 °C). After that, 600 mL of 4 M sodium hydroxide aqueous solution was added dropwise to adjust the pH to 10. The aqueous phase obtained was extracted with ethyl acetate (50 mL × 3), and the organic phases obtained were pooled and washed with saturated sodium chloride solution (50 mL) once, then dried with anhydrous sodium sulfate, and concentrated under reduced pressure to obtain 3.75 g of yellow solid (the intermediate F) in a yield of 80.5%. The solid was directly used for the next reaction. LC-MS (ESI): m/z 307 (M+H)⁺.

The starting intermediate F (3.75 g) was suspended in 19% hydrochloric acid (30 mL) in a 250 mL three-necked reaction flask. The reaction system was heated to 90 °C (the inner temperature) for reaction for 3 hours. When the reaction completed as confirmed with TLC detection, the flask was placed in an ice-salt bath, cooling to below 5 °C. 4 M sodium hydroxide solution (45 mL, 30 eq) was added dropwise to adjust the pH to 10. The aqueous phase obtained was extracted with ethyl acetate (80 mL × 5), and the organic phases obtained were pooled and washed with saturated sodium chloride solution (50 mL) once, then dried with anhydrous sodium sulfate, and concentrated under reduced pressure to obtain 2.45 g of a crude product. The crude product was purified by column chromatography using DCM as an eluent, and then 1.93 g of yellow solid (Compound I) was obtained with a yield of 76%. The total yield of the two steps was found to be 61.2%.

MS (ESI): m/z 177 (M+H)+.

¹H NMR (400 MHz, DMSO) δ 6.76 (d, J = 8.7 Hz, 1H), 6.68 (s, 2H), 6.42 (d, J = 8.7 Hz, 1H), 4.17 (s, 2H), 2.55 (t, J = 5.9 Hz, 2H), 2.46 (t, J = 6.2 Hz, 2H), 2.00 - 1.80 (m, 2H).

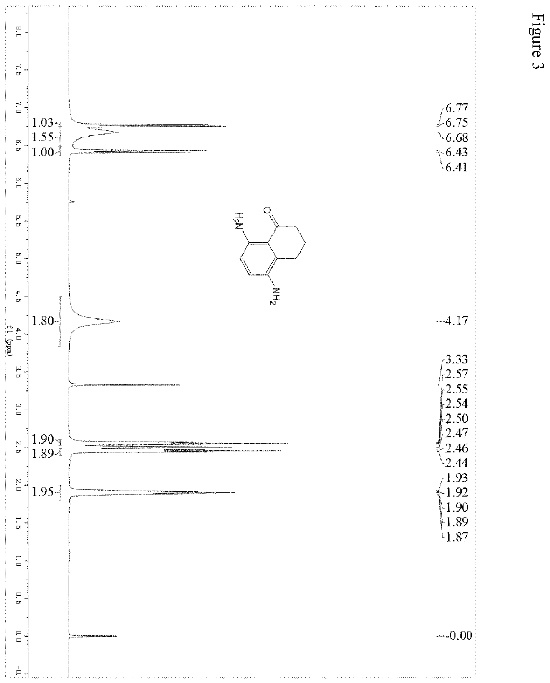

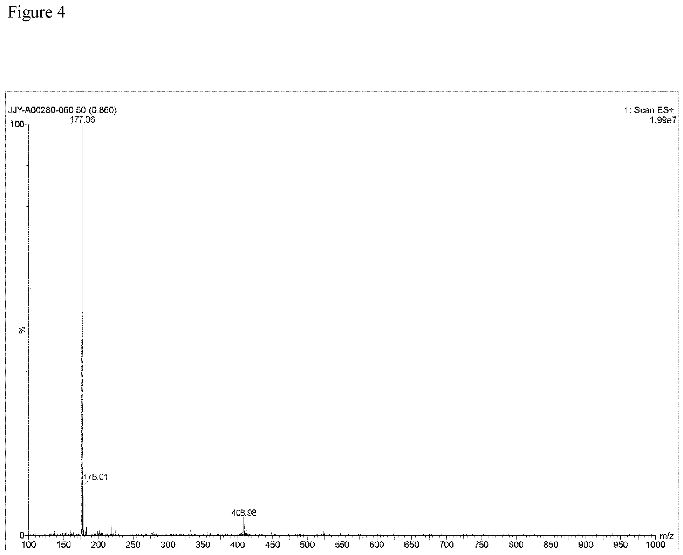

¹H-NMR spectrum and mass spectrum are shown in Figure 3 and Figure 4 respectively.

### Example 7 Synthesis of Compound I (Ring-closing step-by-step)

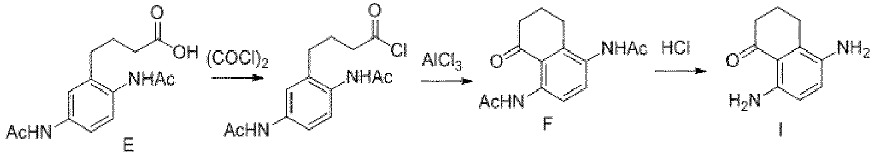

The intermediate E (13.9, 0.05 mol) was dissolved in dichloromethane (200 mL) in a 250 mL three-neck flask, which was placed in an ice bath to control the temperature at 5-10 °C. DMF (1 mL) was added, and then oxalyl chloride (7.62 g, 0.06 mol, 1.2 eq) was added dropwise too. After that, the temperature was maintained for reaction for 2 hours. The reaction liquid was sampled and with methanol added, was detected through TLC to see whether the reaction completed. When the reaction completed, the organic solvent was distilled off under reduced pressure, and the residue obtained was dissolved in methylene chloride (50 mL), and then was dried under reduced pressure again. The dissolving and drying were repeated twice, to obtain 16.2 g of acyl chloride crude product. The crude product was directly used for the next reaction.

Dichloromethane (200 mL) and aluminum trichloride (8 g, 0.06 mol, 1.2 eq) were mixed and stirred in a 250 mL three-neck flask, which was then placed in an ice bath, cooling to 5-10 °C. A solution of the above acyl chloride in dichloromethane (the 16.2 g of crude product was dissolved in 100 mL of dichloromethane) was added dropwise. After that, the temperature was maintained for reaction for 5 hours. When the reaction completed as confirmed with TLC detection, the reaction liquid was poured into 1 N ice-cold hydrochloric acid (300 mL), and stirred for 15 min. Separated phases were obtained, and the aqueous phase obtained was extracted with dichloromethane (100 mL × 3); and the organic phases obtained were pooled and washed with saturated sodium chloride solution (80 mL), then dried with anhydrous sodium sulfate, and concentrated under reduced pressure to obtain 10 g of the intermediate F crude product, part of which was a product without acetyl. The crude product was used in the next reaction without further purification. LC-MS (ESI): m/z 261 (M+H)⁺.

The intermediate F crude product was reacted with hydrochloric acid in the same process as that in Example 6, to obtain 2.3 g of Compound I. The total yield of the three steps was found to be 26.1%.

LC-MS (ESI): m/z 177 (M+H)⁺.

The total yields of Compound I are shown in Table 1.

**Table 1. Total yields of the synthesis of Compound I**

| Route | Compound D | Compound E | Compound I (by polyphosphoric acid) | Total yield |
|---|---|---|---|---|
| Palladium catalyzed coupling | 83.7%* | 78% | 61.2% | 40.0% |
| with zinc reagent | | | | |
| Amide hydrolysis | 87.8% | | | 41.9% |

| | | | | |
|---|---|---|---|---|
| * The yield achieved in Example 1 | | | | |

The above description of the embodiments of the present invention is not intended to limit the present invention, and those skilled in the art may make various changes and modifications to the present invention without departing from the spirit of the present invention, which should fall within the scope of the appended claims.

## Claims

1. A synthetic method of 5,8-diamino-3,4-dihydro-2H-1-naphthalenone (Compound I), the synthetic method comprising: subjecting 2,5-di-protected aminophenylbutyric acid of formula III to a Friedel-crafts reaction to achieve ring closing, and removing the protecting groups on the amino groups to obtain Compound I, 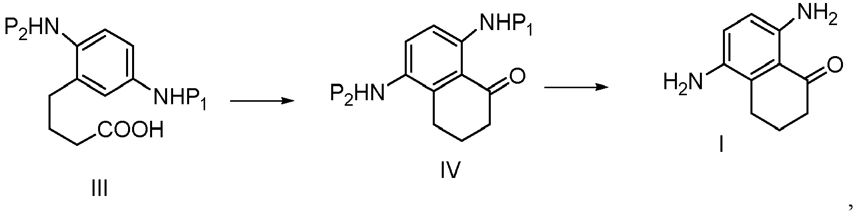 wherein P₁ and P₂ are amino protecting groups.

2. The synthetic method according to claim 1, wherein P₁ and P₂ are independently selected from the group consisting of acetyl, benzoyl, benzyloxycarbonyl (Cbz) and fluorenylmethyloxycarbonyl (Fmoc);
preferably, P₁ and P₂ are the same;
further preferably, both P₁ and P₂ are acetyl.

3. The synthetic method according to claim 1 or 2, wherein the Friedel-crafts reaction is carried out under the catalysis by polyphosphoric acid; or
the Friedel-crafts reaction is carried out by reacting 2,5-di-protected aminophenylbutyric acid of formula III with oxalyl chloride or sulfoxide chloride to prepare an acyl chloride intermediate and then performing catalysis by aluminum trichloride.

4. The synthetic method according to any one of claims 1 to 3, wherein 2,5-di-protected aminophenylbutyric acid of formula III is provided via the following synthetic route 1:
synthetic route 1: 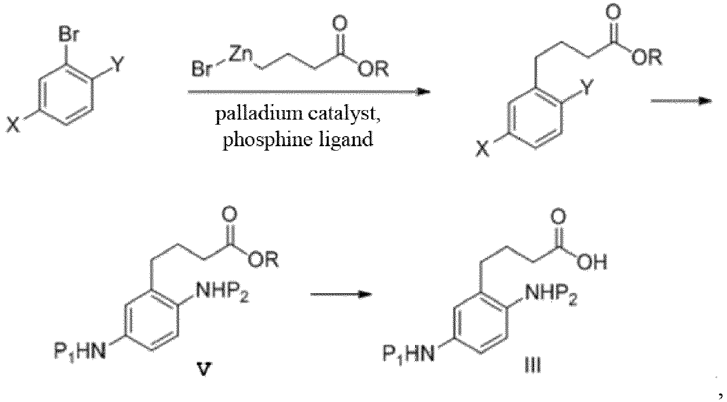
wherein X and Y are independently selected from the group consisting of nitro, amino, or P₁ or P₂ protected amino; R is a C₁-C₆ alkyl or benzyl, preferably selected from the group consisting of methyl, ethyl, isopropyl, tert-butyl and benzyl, more preferably methyl or ethyl;
preferably, the palladium catalyst is selected from the group consisting of palladium acetate, palladium chloride, dppf palladium dichloride, Tetrakis(triphenylphosphine)palladium, and the like, more preferably palladium acetate;
preferably, the phosphine ligand is selected from the group consisting of BINAP, S-PHOS and X-PHOS, more preferably S-PHOS.

5. The synthetic method according to claim 4, wherein synthetic route 1 comprises:
1) when X is amino and Y is nitro: 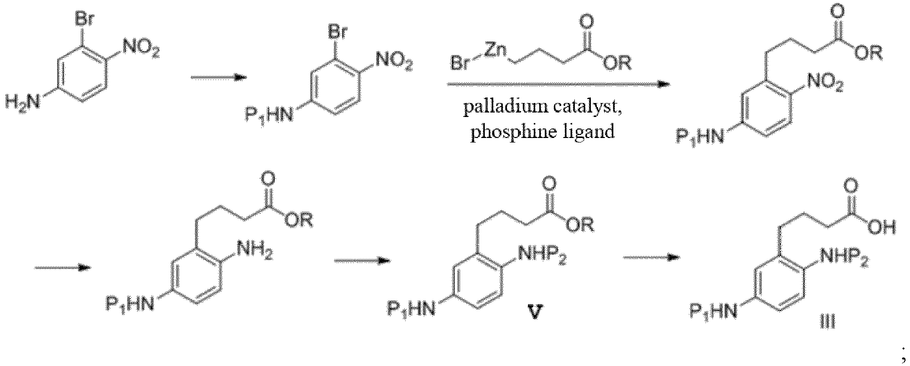
2) when both X and Y are amino: 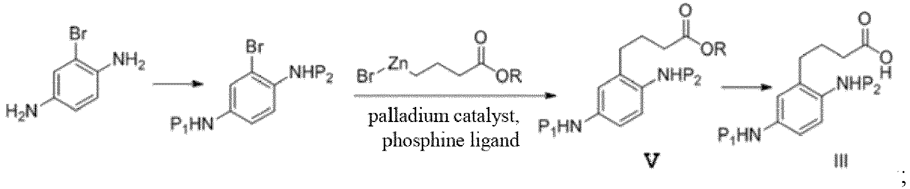
3) when X is nitro and Y is amino:

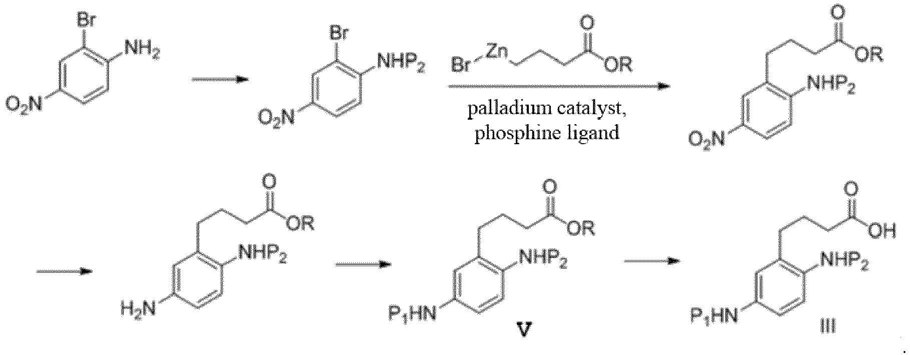

6. The synthetic method according to any one of claims 1 to 3, wherein 2,5-di-protected aminophenylbutyric acid of formula III is provided via the following synthetic route 2:
synthetic route 2: 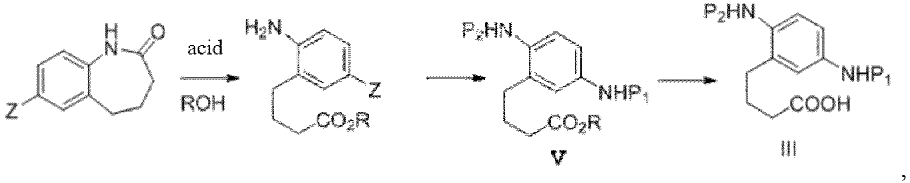
wherein Z is nitro, amino, or P₁ or P₂ protected amino; R is a C₁-C₆ alkyl or benzyl, preferably selected from the group consisting of methyl, ethyl, isopropyl, tert-butyl and benzyl, more preferably methyl or ethyl;
preferably, the acid is selected from the group consisting of sulfuric acid, hydrogen chloride, sulfoxide chloride and p-toluenesulfonic acid, more preferably sulfuric acid.

7. The synthetic method according to claim 6, wherein synthetic route 2 comprises:
1) when Z is amino: 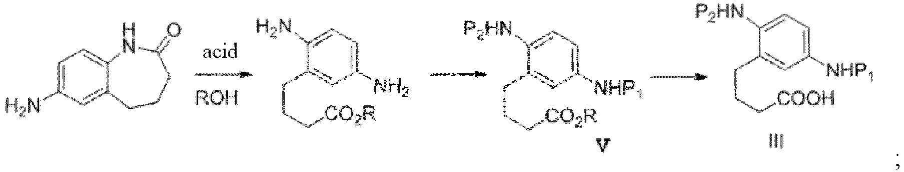
2) when Z is nitro: 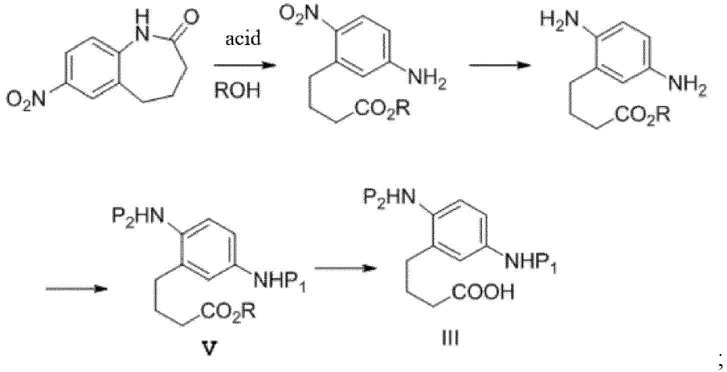 or

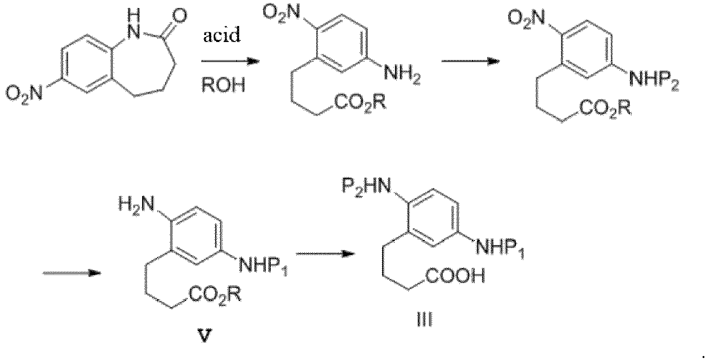

8. A compound of formula V: 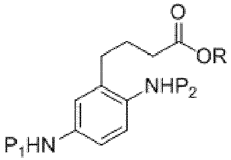 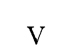 wherein P₁ and P₂ are amino protecting groups; and R is a C₁-C₆ alkyl or benzyl.

9. The compound according to claim 8, wherein P₁ and P₂ are independently selected from the group consisting of acetyl, benzoyl, benzyloxycarbonyl (Cbz) and fluorenylmethyloxycarbonyl (Fmoc); more preferably, P₁ and P₂ are the same; further preferably, both P₁ and P₂ are acetyl;
preferably, R is selected from the group consisting of methyl, ethyl, isopropyl, tert-butyl and benzyl, more preferably methyl or ethyl.

10. The compound according to claim 8 or 9, wherein the compound of formula V 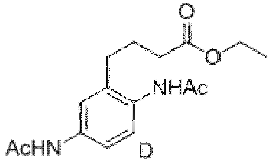 is compound D:

11. A compound of formula III: 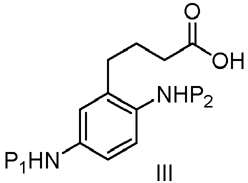 wherein, P₁ and P₂ are amino protecting groups.

12. The compound according to claim 11, wherein P₁ and P₂ are independently selected from the group consisting of acetyl, benzoyl, benzyloxycarbonyl (Cbz) and fluorenylmethyloxy- carbonyl (Fmoc); more preferably, P₁ and P₂ are the same; further preferably, both P₁ and P₂ are acetyl.

13. The compound according to claim 11 or 12, wherein the compound of formula III 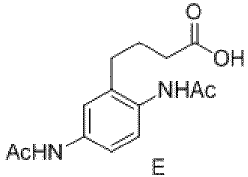 is compound E:

14. Use of the compound of formula V according to any one of claims 8 to 10 and/or the compound of formula III according to any one of claims 11 to 13 for the preparation of Compound I, camptothecins and/or an antibody-drug conjugate.
